# EUROPEAN PATENT APPLICATION

(11) **EP 0 778 021 A1**
(43) Date of publication of application: **11.06.1997**
(21) Application number: 95931411.3
(22) Date of filing: 14.09.1995
(51) Int. Cl.: A61K 31/185, A61K 33/14, A61K 33/00, A61K 33/06, A61K 31/70, A61K 9/08

(54) **EYE DROPS FOR REPAIRING CORNEAL DISTURBANCE**

(30) Priority: 14.09.1994 JP 219917/94
(71) Applicant: TAISHO PHARMACEUTICAL CO. LTD, Tokyo 171 (JP)
(72) Inventor: OHUCHI, Junko Taisho Pharmaceutical Co., Ltd., Tokyo 171 (JP); KATO, Muneyoshi Taisho Pharmaceutical Co., Ltd., Tokyo 171 (JP)
(74) Representative: Weisert, Annekäte, Dipl.-Ing. Dr.-Ing.
(86) International application number: JP9501830
(87) International publication number: WO9608244

(57) **Abstract**

[Object] To provide eye drops which have an high repairing effect on corneal damage and are safe even when applied repeatedly.
[Constitution] Eye drops for repairing corneal damage comprising (a) sodium chloride, potassium chloride and sodium bicarbonate and (b) 0.5 to 3 % by weight of taurine, and having a pH of 5.5 to 8.0 and an osmotic pressure of 250 to 450 mOsm.

## Description

### Technical Field

The present invention relates to eye drops directed to repairing corneal damage comprising specific inorganic salts, taurine, etc.

### Background Art

Corneal damage is caused by wearing contact lenses, ultraviolet rays, dry eye, etc., and has been repaired by using artificial tear fluids containing boric acid, sodium chloride, potassium chloride, calcium chloride. etc. in the past.

However, such prior art is insufficiently effective for repairing corneal damage or has a problem of safety.

An object of the present invention is to provide eye drops which have a high repairing effect on corneal damage and are safe even when applied repeatedly.

### Disclosure of the Invention

As a result of extensive research, the present inventors have found that eye drops comprising specific inorganic salts and taurine enhance synergistically an repairing effect on corneal damage by sodium bicarbonate and taurine, and thereby have accomplished the present invention.

The present invention relates to eye drops for repairing corneal damage comprising (a) sodium chloride, potassium chloride and sodium bicarbonate and (b) 0.5 to 3 % by weight of taurine, and having a pH of 5.5 to 8.0 and an osmotic pressure of 250 to 450 mOsm.

According to the present invention, if the eye drops do not include the above-mentioned ingredient of either (a) or (b), or are not within the above-mentioned ranges of the pH and the osmotic pressure, then they cannot achieve the effect of the present invention.

In addition, it is preferable to contain glucose, besides the above-mentioned ingredients of (a) and (b), in order to further enhance the effect of the present invention. Preferably, the pH ranges from 6.5 to 7.5, and the osmotic pressure ranges from 250 to 350 mOsm, and most preferably, the pH is 7.4, and the osmotic pressure is 286 mOsm.

The eye drops of the present invention can be easily prepared by dissolving the above-mentioned ingredient (a) containing the inorganic salts and the ingredient (b), preferably together with glucose in such an amount rate that the osmotic pressure ranges from 250 to 450 mOsm in sterile purified water, and then adjusting to pH 5.5 to 8.0 with a pH modulator (e.g. sodium borate, citric acid, sodium citrate, hydrochloric acid or sodium hydroxide).

The eye drops of the present invention can contain, besides the above-mentioned essential ingredients, if desired, various components or other effective ingredients usable for ordinary eye drops, for example, anti-inflammatory agents (e.g. dipotassium glycyrrhizinate, ε-aminocaproic acid, allantoin, berberine chloride, berberine sulfate, sodium azulene sulfonate, zinc sulfate, zinc butyrate, lysozyme chloride, etc.), antihistaminic agents (e.g. diphenhydramine hydrochloride, chlorophenilamine maleate, etc.), hyperemia-releasing agents (e.g. naphazoline hydrochloride, tetrahydrozoline hydrochloride, phenylephrine hydrochloride, etc.), vitamins [e.g. an activated vitamin B₂ (flavin adenine dinucleotide sodium), vitamin B₆ (pyridoxine hydrochloride), vitamin B₁₂ (cyanocobalamin), vitamin A acetate (retinol acetate), vitamin E acetate (tocopherol acetate), panthenol, calcium pantothenate, sodium pantothenate, etc.], amino acids (e.g. magnesium potassium L-aspartate, potassium L-aspartate, magnesium L-aspartate, sodium chondroitin sulfate, etc.), refrigerants (e.g. menthol, borneol, camphor, mentha oil, etc.), high polymer additives (e.g. a polyhydric alcohol, polyvinyl alcohol, polyvinylpyrrolidone, methyl cellulose, hydroxyethyl cellulose, hydroxypropylmethyl cellulose, etc.), stabilizers (e.g. ethylenediamine tetraacetate, etc.), preservatives (e.g. benzalkonium chloride, methyl paraben, sorbic acid, etc.), sulfa drugs, etc. The amounts of them are not so much as to degrade the effect of the present invention.

### Industrial Utilizability

The eye drops of the present invention act synergistically on corneal damage by sodium bicarbonate and taurine. In addition, the eye drops are safe even when applied repeatedly, because they do not contain any stimulative ingredients to eyes such as boric acid. Accordingly, the present invention makes it possible to provide the eye drops useful for repairing corneal damage.

The excellent effect of the present invention is illustrated by the following test example.

### Test example

The measurement of the amount of LDH in tear fluid is reported to be useful for the judgement of the grade of corneal damage in Chem. Pharm. Bull., vol. 41(2), pp. 335 - 338, 1993.

### (Test method)

(1) Irradiation of ultraviolet rays to rabbit cornea
   Eight rabbits (Japanese white rabbit, male, 5-month-old, weighing 2.4 - 3.0 kg) were fixed, and ultraviolet rays (UV-B) were irradiated to the cornea for 10 minutes (750 - 800 µW/cm²).
(2) The eye drops prepared in Example 2 were topically applied to the right eye for 5 days (3 drops per once, 4 times per day). The left eye was untreated.
(3) Measurement of lactate dehydrogenase (LDH)
   The amount of LDH release was measured before and immediately after the irradiation of ultraviolet rays, and on 1 day to 5 days after the irradiation. 250 µl of saline was topically applied to the eye and pooled for one minute, and then 50 µl of the sample solution was measured by using an automatic analyzer (7150-type, manufactured by Hitachi Co.).

### (Results)

Results are shown in Table 1. The amount of LHD release was found to be decreased by the topical application of the eye drops prepared in Example 2 on 2 days and 4 days after the irradiation of ultraviolet rays. That is, the total amount of LHD release in the period from immediately after the irradiation of ultraviolet rays to 5 days after the irradiation was significantly decreased by the topical application of the eye drops prepared in Example 2. It is established from the above fact that the eye drops of the present invention have a repairing effect on the corneal damage.

**Table 1**

| Amount of LDH released from rabbit cornea (unit: IU/L) 〈Right eye: treated-eye group〉 | |
|---|---|
| before irradiation | 29 |
| immediately after irradiation | 26 |
| after one day | 145 |
| after 2 days | 153 |
| after 4 days | 35 |

| 〈Left eye: untreated-eye group〉 | |
|---|---|
| before irradiation | 27 |
| immediately after irradiation | 29 |
| after one day | 150 |
| after 2 days | 263 |
| after 4 days | 71 |

### Best Mode for Carrying Out the Invention

The present invention is illustrated in more detail by the following examples.

### Example 1

1000 mg of taurine, 557 mg of sodium chloride, 106 mg of potassium chloride, 11 mg of calcium chloride and 15 mg of magnesium sulfate were together dissolved in 90 ml of sterile purified water, and adjusted to pH 7.4 with sodium borate. The total volume of the solution was made up to 100 ml by adding sterile purified water to give eye drops of which osmotic pressure is 286 mOsm as a result of the measurement.

### Example 2

1000 mg of taurine, 480 mg of sodium chloride, 91 mg of potassium chloride and 151 mg of sodium bicarbonate were together dissolved in 90 ml of sterile purified water, and adjusted to pH 7.4 with hydrochloric acid. The total volume of the solution was made up to 100 ml by adding sterile purified water to give eye drops of which osmotic pressure is 286 mOsm as a result of the measurement.

### Example 3

1000 mg of taurine, 542 mg of sodium chloride, 103 mg of potassium chloride, 11 mg of calcium chloride, 14 mg of magnesium sulfate and 100 mg of glucose were together dissolved in 90 ml of sterile purified water, and adjusted to pH 7.4 with sodium borate. The total volume of the solution was made up to 100 ml by adding sterile purified water to give eye drops of which osmotic pressure is 286 mOsm as a result of the measurement.

### Example 4

1000 mg of taurine, 467 mg of sodium chloride, 89 mg of potassium chloride, 146 mg of sodium bicarbonate and 100 mg of glucose were together dissolved in 90 ml of sterile purified water, and adjusted to pH 7.4 with hydrochloric acid. The total volume of the solution was made up to 100 ml by adding sterile purified water to give eye drops of which osmotic pressure is 286 mOsm as a result of the measurement.

### Example 5

1000 mg of taurine, 472 mg of sodium chloride, 90 mg of potassium chloride, 9 mg of calcium chloride, 12 mg of magnesium sulfate and 148 mg of sodium bicarbonate were together dissolved in 90 ml of sterile purified water, and adjusted to pH 7.4 with hydrochloric acid. The total volume of the solution was made up to 100 ml by adding sterile purified water to give eye drops of which osmotic pressure is 286 mOsm as a result of the measurement.

### Example 6

1000 mg of taurine, 459 mg of sodium chloride, 87 mg of potassium chloride, 9 mg of calcium chloride, 12 mg of magnesium sulfate, 144 mg of sodium bicarbonate and 100 mg of glucose were together dissolved in 90 ml of sterile purified water, and adjusted to pH 7.4 with hydrochloric acid. The total volume of the solution was made up to 100 ml by adding sterile purified water to give eye drops of which osmotic pressure is 286 mOsm as a result of the measurement.

### Example 7

1000 mg of taurine, 397 mg of sodium chloride, 75 mg of potassium chloride and 125 mg of sodium bicarbonate were together dissolved in 90 ml of sterile purified water, and adjusted to pH 7.4 with hydrochloric acid. The total volume of the solution was made up to 100 ml by adding sterile purified water to give eye drops of which osmotic pressure is 250 mOsm as a result of the measurement.

### Example 8

1000 mg of taurine, 860 mg of sodium chloride, 163 mg of potassium chloride and 271 mg of sodium bicarbonate were together dissolved in 90 ml of sterile purified water, and adjusted to pH 7.4 with hydrochloric acid. The total volume of the solution was made up to 100 ml by adding sterile purified water to give eye drops of which osmotic pressure is 450 mOsm as a result of the measurement.

## Claims

1. Eye drops for repairing corneal damage comprising (a) sodium chloride, potassium chloride and sodium bicarbonate and (b) 0.5 to 3 % by weight of taurine, and having a pH of 5.5 to 8.0 and an osmotic pressure of 250 to 450 mOsm.

2. Eye drops for repairing corneal damage comprising (a) sodium chloride, potassium chloride and one or more inorganic salts selected from the group consisting of sodium bicarbonate, magnesium sulfate and calcium chloride, (b) 0.5 to 3 % by weight of taurine and (c) glucose, and having a pH of 5.5 to 8.0 and an osmotic pressure of 250 to 450 mOsm.
